# EUROPEAN PATENT SPECIFICATION

(11) **EP 2 968 319 B1**
(45) Date of publication and mention of the grant of the patent: **08.01.2020**
(21) Application number: 14770876.2
(22) Date of filing: 14.03.2014
(51) Int. Cl.: A61P 25/28, A61K 9/00, A61K 31/44, A61K 31/175

(54) **TREATMENT FOR CHEMOTHERAPY-INDUCED COGNITIVE IMPAIRMENT**
BEHANDLUNG VON DURCH CHEMOTHERAPIE AUSGELÖSTE KOGNITIVE BEEINTRÄCHTIGUNG
TRAITEMENT D'UN DÉFICIT COGNITIF INDUIT PAR UNE CHIMIOTHÉRAPIE

(30) Priority: 14.03.2013 US 201313803842
(43) Date of publication of application: 20.01.2016
(73) Proprietor: Ghanbari, Hossein, Potomac, MD 20854 (US)
(72) Inventor: GHANBARI, Hossein, A., Potomac, MD 20854 (US); JIANG, Zhi-gang, Gaithersburg, MD 20878 (US)
(74) Representative: FRKelly
(86) International application number: PCT/US2014/028039
(87) International publication number: WO 2014/152864

(56) References cited:
- WO-A1-2012/149267
- US-A- 6 114 376
- US-A1- 2006 160 826
- US-A1- 2006 194 810
- US-A1- 2007 243 132
- US-A1- 2008 039 471
- US-A1- 2009 275 587
- US-A1- 2009 286 799
- US-A1- 2010 120 763
- US-B2- 7 456 179
- US-B2- 8 367 675
- ZHI-GANG JIANG ET AL: "Neuroprotective Activity of 3-Aminopyridine-2-Carboxaldehyde Thiosemicarbazone (PAN-811), a Cancer Therapeutic Agent", CNS DRUG REVIEWS., vol. 12, no. 1, 1 March 2006 (2006-03-01), pages 77-90, XP055301421, US ISSN: 1080-563X, DOI: 10.1111/j.1527-3458.2006.00077.x
- GREGORY W KONAT ET AL: "Cognitive dysfunction induced by chronic administration of common cancer chemotherapeutics in rats", METABOLIC BRAIN DISEASE, KLUWER ACADEMIC PUBLISHERS-PLENUM PUBLISHERS, NE, vol. 23, no. 3, 9 August 2008 (2008-08-09) , pages 325-333, XP019611556, ISSN: 1573-7365
- ZHI-GANG JIANG ET AL: "PAN-811 Blocks Chemotherapy Drug-Induced In Vitro Neurotoxicity, While Not Affecting Suppression of Cancer Cell Growth", OXIDATIVE MEDICINE AND CELLULAR LONGEVITY, vol. 61, no. 11, 1 January 2016 (2016-01-01), pages 1096-11, XP055301425, US ISSN: 1942-0900, DOI: 10.1016/s0065-2571(98)00017-x
- JIANG Z -G ET AL: "PAN-811 prevents chemotherapy-induced cognitive impairment and preserves neurogenesis in the hippocampus of adult rats", PLOS ONE 20180101 PUBLIC LIBRARY OF SCIENCE USA, vol. 13, no. 1, 1 January 2018 (2018-01-01), ISSN: 1932-6203

## Description

### BACKGROUND OF THE INVENTION

Chemotherapy has improved survival rates in patients with many of the common cancers. However, one of the most common complications of chemotherapeutic drugs is toxicity to the central nervous system (CNS), named chemotherapy-induced cognitive impairment, chemotherapy-induced cognitive dysfunction, post- chemotherapy cognitive impairment (PCCI), chemo fog, or chemo brain. Chemo brain can be very frustrating both for those who are living with cancer, and their loved ones who are trying to support them. Chemo brain can seriously affect quality of life and life itself in cancer patients. This toxicity can manifest in many ways, including encephalopathy syndromes and confusional states, seizure activity, headache, cerebrovascular complications and stroke, visual loss, cerebellar dysfunction, and spinal cord damage with myelopathy. There is reliable evidence that, as a result of treatment, a subset of cancer survivors experience cognitive problems that can last for many years after the completion of chemotherapy. These include attention deficits, memory loss, and confused thought processes. Up to 70% of patients report that their cognitive difficulties persist well beyond the duration of treatment. Studies that have measured cognitive function using standardized neuropsychological assessments have found mild to moderate effects of chemotherapy on cognitive performance in 15-50% of the survivors after treatment. Longitudinal studies have shown that, in a subset of survivors, cognitive difficulties can persist for between 1 and 2 years after the completion of chemotherapy. Cross-sectional studies have found cognitive impairments lasting between 4 and 10 years after chemotherapy. Although recent prospective studies show that about 20% of cancer patients experience cognitive dysfunction even before chemotherapy, chemotherapy agents produce significant cognitive impairment in laboratory animals that are free from cancer as well as from other treatment- and diagnosis-related factors. Healthy rodents that are given chemotherapy show increase in cell death in the central nervous system, increase in oxidative stress, increase in microglia activity, suppression of hippocampal neurogenesis, decreases in levels of neurotrophic factors, and decreases in levels of hippocampal catecholamines, as compared to baseline values. The etiology of chemotherapy-induced cognitive impairment is largely unknown, but several candidate mechanisms have been suggested, including oxidative stress, impaired blood-brain barrier (BBB), neuroinflammation, decreased neurogenesis, etc.

Oxidative stress plays a key role in cognitive disorders caused by certain types of anticancer drugs, such as antimetabolites, mitotic inhibitors, topoisomerase inhibitors and paclitaxel, etc. These chemotherapeutic agents are not known to rely on oxidative mechanisms for their anticancer effects. Among the antimetabolite drugs, methotrexate (MTX), 5-fluorouracil (5-FU, a widely used chemotherapeutic agent), and cytosine arabinoside are most likely to cause CNS toxicity. 5-FU can cause both acute and delayed neurotoxicity. Acute neurotoxicity manifests as encephalopathy cerebellar syndrome or as seizures. Acute neurotoxicity due to 5-FU is dose-related and generally self-limiting. 5-FU readily crosses the blood-brain barrier and disrupts cell proliferation. Clinically relevant concentrations of 5-FU were toxic for both central nervous system (CNS) progenitor cells and non-dividing oligodendrocytes in both *in vitro* and *in vivo.* Short-term systemic administration of 5-FU caused both acute CNS damage and a syndrome of progressively worsening delayed damage to myelinated tracts of the CNS associated with altered transcriptional regulation in oligodendrocytes and extensive myelin pathology. Functional analysis also provided the first demonstration of delayed effects of chemotherapy on the latency of impulse conduction in the auditory system, offering the possibility of non-invasive analysis of myelin damage associated with cancer treatment. Delayed neurotoxicity has been reported when fluorouracil was given in combination with levamisole; this form of subacute multifocal leukoencephalopathy is immune mediated. Although no report for 5-FU to increase CNS oxidative stress has been found yet, it has been indicated inducing apoptosis in rat cardiocytes through intracellular oxidative stress, increasing oxidative stress in the plasma of liver cancer patients, and decreasing glutathione in bone marrow cells. Another antimetabolite MTX can cross the blood-brain barrier as well. It resulted in an increase of oxidative stress in cerebral spinal fluid and executive dysfunction in MTX -treated patients of pediatric acute lymphoblastic leukemia. A recent observation also indicates that genetic polymorphism for methionine is a potent risk factor for MTX -induced central nervous system toxicity.

Oxidative stress could be a common path for chemotherapy-induced cognitive impairment and neurodegenerative diseases. Oxidative stress can cause single and double DNA strand breaks and is the most frequent cause of DNA damage in neuronal cells. Oxidative damage can occur through exposure to foreign agents or result from an endogenous mechanism. Oxidative damage has been associated with numerous neurodegenerative diseases such as Alzheimer's disease and Parkinson's. Patients displaying mild cognitive impairment exhibit higher levels of oxidative DNA damage in both peripheral leukocytes and the brain. Many chemotherapeutic agents take advantage of the DNA damaging effects of oxidative stress; however, the effects of oxidative stress are not confined to abnormal cells. Evidence of oxidative damage has been seen in peripheral blood lymphocytes in breast cancer patients treated with chemotherapy. In addition, chemotherapy patients displayed decreased DNA repair abilities. Chemotherapy treatment is associated with increased levels of nonprotein bound iron, increased levels of free radicals, and decreased antioxidant capacity, all factors suggested to increase oxidative stress. It is proposed that MTX treatment inhibits protective factors that may prevent radical damage. As a result, polyunsaturated fatty acid chains within the cell membranes are more susceptible to attack by reactive oxygen species. These initial attacks signal other lipid peroxy radicals to form, triggering a cascade of cell membrane damage.

Currently there are no proven treatments for chemotherapy-induced cognitive impairment. Some efforts have been focused on correcting cognitive deficits rather blocking neurotoxic pathway that was induced with chemotherapeutical drug. These include erythropoietin (a glycoprotein to stimulate the production of red blood cells), methylphenidate (modulating catecholaminergic tone), modafinil (releasing catecholamines, norepinephrine, dopamine and histamine), donepezil (a cholinesterase inhibitor), and fluoxetine (a selective serotonin reuptake inhibitor). In contrast to above, antioxidative treatment would be a promising strategy for the treatment. Consumption of foods high in antioxidants and antioxidant supplementation appear to slow the rate of cognitive decline associated with aging and disease in humans and rodents. Several preclinical studies have shown that antioxidant treatment prevents chemotherapy-induced oxidative stress and cognitive deficits when administered prior to and during chemotherapy. For example, systemic treatment in healthy mice with γ-glutamyl cysteine ethyl ester prior to doxorubicin treatment significantly decreased markers of oxidative stress, namely, protein oxidization and lipid peroxidization. However, behavior was not assessed in this study. Prior intracerebroventricular treatment with the antioxidant zinc sulfate (ZnSO4) prevented short-term memory impairments induced by systemic carmustine (BCNU) treatment. Specifically, BCNU treatment caused rats to make more errors during learning and recall of the radial arm maze, whereas treatment with ZnSO4 prior to BCNU prevented these deficits in learning and memory. In addition, hippocampal cell death and inflammation induced by BCNU treatment were prevented in rats pretreated with ZnSO4. Another case for reduction of anticancer drug-caused cognitive deficit with antioxidant is that there was no short-term memory impairment when rats that received cyclophosphamide and doxorubicin were treated with the antioxidant *N*-acetyl cysteine during chemotherap. A recent review of 29 placebo-controlled randomized control trials of cancer survivors with deficits confirmed, by comparing psychometric tests, that the greatest benefits of *Ginkgo biloba* were found in executive functioning, selective attention, and memory.

Taken together, this information suggests that treatment with antioxidants prior to and during chemotherapy prevents the occurrence of cognitive deficits shortly after chemotherapy. Human trials of antioxidant supplementation in patients with breast and lung cancer have demonstrated an increase in survival, although alkylating agents, antitumor antibiotics, and topisomerase II inhibitors depend on the generation of free radicals for their therapeutic action. It was found, by investigating the MEDLINE® and CANCERLIT® databases from 1965 to November 2003, that antioxidants and other nutrients do not interfere with chemotherapy of alkylating agents (e.g. cisplatin), antitumor antibiotics (e.g. doxorubicin) and topoisomerase II inhibitor (e.g. etoposide) or radiation therapy and can increase kill and increase survival. As there are no proven treatments, there is a need for methods to properly treat chemotherapy-induced cognitive impairment. The present invention provides just such a method.

Zhi-Gang Jiang et al discuss neuroprotective activity of 2-aminopyridine-2-carboxaldehyde thiosemicarbazone (PAN-811), a cancer therapeutic agent, in "CNS Drug Reviews" vol. 12(1), 1 March 2006, p. 77-90. Gregory W Konat et al discuss cognitive dysfunction induced by chronic administration of common cancer chemotherapeutics in rats, in "Metabolic Brain Disease" vol. 23(3), 9 August 2008, p. 325-333.

### SUMMARY OF THE INVENTION

The present invention is directed to a composition comprising at least one thiosemicarbazone compound for use in a method for the treatment of anti-cancer therapy induced cognitive impairment, the use comprising the step of administering to a patient the composition comprising the at least one thiosemicarbazone compound (Formula II):

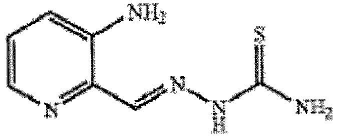

In some embodiments, the step of administering is intravenous, intraperitoneal, subcutaneous, intramuscular, topical, transdermal or oral.

In some embodiments, the composition is an injectable and/or infusible solution.

In some embodiments, the composition is formulated as a micro emulsion. In some embodiments, the composition is formulated as a liposome.

### DETAILED DESCRIPTION OF THE INVENTION

The present invention is directed to a composition comprising at least one thiosemicarbazone compound for use in a method for the treatment of anti-cancer therapy induced cognitive impairment, the use comprising the step of administering to a patient the composition comprising the at least one thiosemicarbazone compound (Formula II):

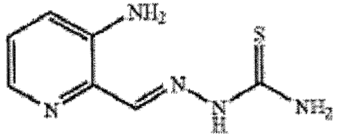

The means for synthesis of thiosemicarbazone compounds are well known in the art. Such synthetic schemes are described in U.S. Pat. Nos. 5,281,715; 5,767,134; 4,447,427; 5,869,676 and 5,721,259. The chemical structures of PAN-811's analogues are shown in U.S. Pat. No 7,456,179, and patent applications of 20090275587, 20060194810 and 20060160826.

The pharmaceutical compositions required by the present invention typically comprise the thiosemicarbazone compound and a pharmaceutically acceptable carrier. As used herein "pharmaceutically acceptable carrier" includes any and all solvents, dispersion media, coatings, antibacterial and antifungal agents, isotonic and absorption delaying agents that are physiologically compatible. The type of carrier can be selected based upon the intended route of administration. In various embodiments, the carrier is suitable for intravenous, intraperitoneal, subcutaneous, intramuscular, topical, transdermal or oral administration. Pharmaceutically acceptable carriers include sterile aqueous solutions or dispersions and sterile powders for the extemporaneous preparation of sterile injectable solutions or dispersion. The use of such media and agents for pharmaceutically active substances is well known in the art. Except insofar as any conventional media or agent is incompatible with the active compound, use thereof in the pharmaceutical compositions of the invention is contemplated. Supplementary active compounds can also be incorporated into the compositions.

Therapeutic compositions typically must be sterile and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyetheylene glycol), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as mannitol, sorbitol, or sodium chloride in the composition. Prolonged absorption of the injectable compositions can be brought about by including in the composition an agent which delays absorption, for example, monostearate salts and gelatin. Moreover, the compounds can be administered in a time release formulation, for example in a composition which includes a slow release polymer. The active compounds can be prepared with carriers that will protect the compound against rapid release, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, polylactic acid and polylactic, polyglycolic copolymers (PLG). Many methods for the preparation of such formulations are generally known to those skilled in the art.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Depending on the route of administration, the compound may be coated in a material to protect it from the action of enzymes, acids and other natural conditions which may inactivate the agent. For example, the compound can be administered to a subject in an appropriate carrier or diluent co-administered with enzyme inhibitors or in an appropriate carrier such as liposomes. Pharmaceutically acceptable diluents include saline and aqueous buffer solutions. Enzyme inhibitors include pancreatic trypsin inhibitor, diisopropylfluoro-phosphate (DEP) and trasylol. Liposomes include water- in-oil-in-water emulsions as well as conventional liposomes. Dispersions can also be prepared in glycerol, liquid polyethylene glycols, and mixtures thereof and in oils. Under ordinary conditions of storage and use, these preparations may contain a preservative to prevent the growth of microorganisms.

The active agent in the composition (i.e., one or more thiosemicarbazones) preferably is formulated in the composition in a therapeutically effective amount. A "therapeutically effective amount" refers to an amount effective, at dosages and for periods of time necessary, to achieve the desired therapeutic result to thereby influence the therapeutic course of a particular disease state. A therapeutically effective amount of an active agent may vary according to factors such as the disease state, age, sex, and weight of the individual, and the ability of the agent to elicit a desired response in the individual. Dosage regimens may be adjusted to provide the optimum therapeutic response. A therapeutically effective amount is also one in which any toxic or detrimental effects of the agent are outweighed by the therapeutically beneficial effects. In another embodiment, the active agent is formulated in the composition in a prophylactically effective amount. A "prophylactically effective amount" refers to an amount effective, at dosages and for periods of time necessary, to achieve the desired prophylactic result. Typically, since a prophylactic dose is used in subjects prior to or at an earlier stage of disease, the prophylactically effective amount will be less than the therapeutically effective amount.

The amount of active compound in the composition may vary according to factors such as the disease state, age, sex, and weight of the individual. Dosage regimens may be adjusted to provide the optimum therapeutic response. For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active compound calculated to produce the desired therapeutic effect in association with the required pharmaceutical carrier. The specification for the dosage unit forms of the invention are dictated by and directly dependent on (a) the unique characteristics of the active compound and the particular therapeutic effect to be achieved, and (b) the limitations inherent in the art of compounding such an active compound for the treatment of sensitivity in individuals.

A compound of the invention can be formulated into a pharmaceutical composition wherein the compound is the only active agent therein. Alternatively, the pharmaceutical composition can contain additional active agents.

3-aminopyridine-2-carboxaldehyde thiosemicarbazone (hereinafter "PAN-811"), with a molecular weight of 195.24 Da, has demonstrated potent neuroprotective activities in several neurodegenerative models. PAN-811 was originally developed for cancer therapy due to its ability to inhibit ribonucleotide reductase, a key enzyme required for DNA synthesis. Our previous studies demonstrated that PAN-811 at concentration of 0.45 µM fully blocked ischemic neurodegeneration and at 1.2µM completely halted hypoxia-induced neuronal cell death (Jiang et al., 2006). PAN-811 was administered intracerebroventricularly (*i.c.v*.) at a dose of 50 µg per rat at 1 h after arterial occlusion. Staining of consecutive brain sections and computer-assisted quantitative analysis demonstrated that PAN-811 reduced the infarct volume by 59% in PAN-811 treated rats. We also investigated the effect of a single intravenous (i.v.) bolus injection of PAN-811. Two-hour middle cerebral artery occlusion (MCAo) with cerebral blood flow reduction of 75% or greater resulted in infarct formation, brain edema and a significant number of premature deaths. PAN-811 treatment reduced infarct volume in a dose dependent manner with a maximal protection of 50% at a dose of 2mg/kg. PAN-811 treatment (2mg/kg) also resulted in a 70% reduction in brain edema volume. Accordingly, the mortality in PAN-811 treated groups was collectively reduced by 44% (Jiang et al, 2008). Mechanistically PAN- 811 not only prevents glutamate-induced excitatory cytotoxicity, veratridine-induced sodium channel opening that is related to Ca²⁺ influx and staurosporine-induced apoptosis, but also blocks oxidative stress-induced neuronal cell death in many ways. PAN-811 at a concentration as low as 1 µM suppressed in vitro hydrogen peroxide- induced LDH release by 78% (with P<0.01, compared to untreated/H₂O₂-insulted group) and at a concentration of 10 µM achieved maximal protection (by 90% comparing with untreated and H₂O₂-insulted group) with an ECso of ∼0.55 µM (Pan et al., 2009). PAN-811 also inhibited oxidative stress-induced cell death of human Alzheimer's disease-derived and age-matched olfactory neuroepithelial cells via suppression of intracellular reactive oxygen species.

Importantly, PAN-811 manifested as a free radical scavenger in a cell free system where PAN-811 reduced 500µM of a stable free radical diphenylpicrylhydrazyl by 70%. Taken together, PAN-811 has manifested as a potent antioxidant and neuroprotectant. Based on the key role of oxidative stress in chemo brain and also the potent antioxidative and neuroprotective effects of PAN-811, we have discovered that PAN-811 is a therapeutic agent for chemotherapy- induced cognitive deficit or chemo brain. PAN-811 should inhibit chemotherapy- induced cognitive deficit that is not only caused with antimetabolites (cytarabine, gludarabine, fluorouracil, mercaptopurine, methotrexate, thioguanine, gemcitabine, hydroxyurea), mitotic inhibitors (vincristine, vinblastine, vinorelbine), topoisomerase inhibitors (topotecan, irenotecan), paclitaxel, docetaxel and asparaginase, but also with alkylating agents (busulfan, carmustine, lomustine, chlorambucil, cyclophosphamide, cisplatin, carboplatin, ifosamide, mechlorethamine, melphalan, thiotepa, dacarbazine, procarbazine), antitumor antibiotics (bleomycin, dactinomycin, daunorubicin, doxorubicin, idarubicin, mitomycin, mitoxantrone, plicamycin), topoisomerase II inhibitor (etoposide, teniposide), and radiation therapy.

## Claims

1. A composition comprising at least one thiosemicarbazone compound for use in a method for the treatment of anti-cancer therapy induced cognitive impairment, the use comprising the step of administering to a patient the composition comprising the at least one thiosemicarbazone compound (Formula II):

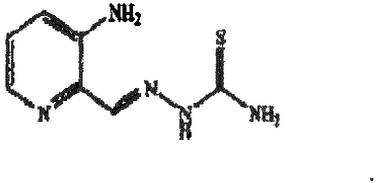

2. The composition for use of claim 1, wherein the step of administering is intravenous, intraperitoneal, subcutaneous, intramuscular, topical, transdermal or oral.

3. The composition for use of claim 1, wherein the composition is an injectable and/or infusible solution.

4. The composition for use of claim 1, wherein the composition is formulated as a micro emulsion.

5. The composition for use of claim 1, wherein the composition is formulated as a liposome.

## Patentansprüche

1. Zusammensetzung, umfassend mindestens eine Thiosemicarbazon-Verbindung zur Verwendung in einem Verfahren zur Behandlung einer kognitiven Beeinträchtigung, die durch eine Anti-Krebs-Therapie induziert ist, wobei die Verwendung den Schritt eines Verabreichens, an einen Patienten, der Zusammensetzung umfasst, die die mindestens eine Thiosemicarbazon-Verbindung (Formel II) umfasst:

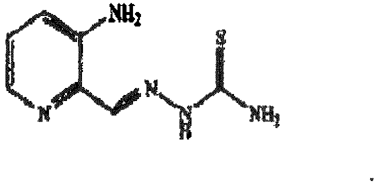

2. Zusammensetzung zur Verwendung nach Anspruch 1, wobei der Schritt des Verabreichens intravenös, intraperitoneal, subkutan, intramuskulär, topisch, transdermal oder oral ist.

3. Zusammensetzung zur Verwendung nach Anspruch 1, wobei die Zusammensetzung eine injizierbare und/oder infundierbare Lösung ist.

4. Zusammensetzung zur Verwendung nach Anspruch 1, wobei die Zusammensetzung als eine Mikroemulsion formuliert ist.

5. Zusammensetzung zur Verwendung nach Anspruch 1, wobei die Zusammensetzung als ein Liposom formuliert ist.

## Revendications

1. Composition comprenant au moins un composé thiosemicarbazone pour une utilisation dans une méthode de traitement d'un déficit cognitif induit par un traitement anti-cancereux, l'utilisation comprenant l'étape d'administration à un patient de la composition comprenant le au moins un composé thiosemicarbazone (Formule II):

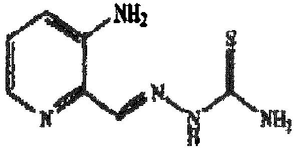

2. Composition pour l'utilisation selon la revendication 1, dans laquelle l'étape d'administration est intraveineuse, intrapéritonéale, sous-cutanée, intramusculaire, topique, transdermique ou orale.

3. Composition pour l'utilisation selon la revendication 1, dans laquelle la composition est une solution injectable et/ou perfusable.

4. Composition pour l'utilisation selon la revendication 1, dans laquelle la composition est formulée sous forme d'une micro émulsion.

5. Composition pour une utilisation selon la revendication 1, dans laquelle la composition est formulée sous forme d'un liposome.
